Europäisches Patentamt

European Patent Office

Office européen des brevets

(11) Veröffentlichungsnummer: **0 387 754**
**A1**

(12) **EUROPÄISCHE PATENTANMELDUNG**

(21) Anmeldenummer: 90104638.3

(51) Int. Cl.⁵ **A61B 17/22**

(22) Anmeldetag: **12.03.90**

(30) Priorität: 17.03.89 DE 8903333 U

(43) Veröffentlichungstag der Anmeldung:
**19.09.90 Patentblatt 90/38**

(84) Benannte Vertragsstaaten:
**AT CH DE FR GB IT LI NL SE**

(71) Anmelder: Schott Glaswerke
**Hattenbergstrasse 10**
**D-6500 Mainz(DE)**

(84) **CH DE FR IT LI NL SE AT**

Anmelder: Carl-Zeiss-Stiftung trading as
**SCHOTT GLASWERKE**
**Hattenbergstrasse 10**
**D-6500 Mainz 1(DE)**

(84) **GB**

(72) Erfinder: **Müller, Gerhard, Prof.-Dr.**
**An der Rehwiese 8**
**D-1000 Berlin 38(DE)**
Erfinder: **Kar, Hasan, Dipl.-Phys.**
**Schildhornstrasse 22**
**D-1000 Berlin 41(DE)**
Erfinder: **Dörschel, Klaus, Dr.**
**Himbeersteig 16**
**D-1000 Berlin 38(DE)**
Erfinder: **Schönborn, Karl-Heinz, Dr.**
**Konrad-Adenauer-Strasse 27**
**D-6500 Mainz 41(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Kathetersystem zur Uebertragung von Laserstrahlung in Gefaesssysteme des menschlichen Koerpers.**

(57) In einem Kathetersystem zur Übertragung von Laserstrahlung in Gefäßsysteme des menschlichen Körpers sind die einzelnen Lichtleitfasern (3) am distalen Lichtaustrittsende des Ringkatheters unter Vergrößerung seines Umfangs aufweitbar ausgebildet, so daß damit auch Ablagerungen im Gefäßsystem, die im wesentlichen außerhalb des Durchmessers des Ringkatheters liegen, wirksam beseitigt werden können. Durch mit Druckfluid beaufschlagbare Dehnkammereinrichtungen oder vorzugsweise mittels eines in den Innnenkanal des Ringkatheters einführbaren Miniaturdilatationskatheters (9) kann der Abstand zwischen den einzelnen Lichtleitfasern des Lichtleiterkranzes oder zwischen bestimmten Umfangsgruppen von Lichtleitfasern des Kranzes am distalen Ende vergrößert werden. Dabei lassen sich diese Umfangsgruppen über die Koordination von Bereichsblenden an der hexagonal dichtesten Packung der Lichtleitfasern am proximalen Ende des Katheters selektiv mit Laserlicht beaufschlagen.

Fig. 1

EP 0 387 754 A1

Die Erfindung betrifft ein Kathetersystem zur Übertragung von Laserstrahlung zu Behandlungszwecken, insbesondere in Gefäßsystemen des menschlichen Körpers nach dem Oberbegriff des Patentanspruches 1. Es ist bekannt, daß sich bestimmte Ablagerungen in Gefäßsystemen, beispielsweise des menschlichen Körpers, insbesondere in Adern, durch Einwirkung von Laserstrahlung bestimmter Energiedichte, wie vermutet wird, in Folge des fotohydraulischen Effektes ablösen und die behandelten Gefäße somit rekanalisieren lassen. Ein Kathetersystem für derartige Anwendungen ist beispielsweise in der DE-OS 3 739 965 beschrieben.

Vorzugsweise weisen solche Kathetersysteme einen Ringkatheter mit einem Innenkanal auf, um den herum in dem Wandaufbau des Katheters ein Kranz von Lichtleitfasern für den Transport des Laserlichtes zum Behandlungsort am distalen Ende des Katheters angeordnet ist.

Bei der Ausbildung eines solchen Ringkatheters kommt es darauf an, daß das aus den ringförmig angeordneten Lichtleitfasern austretende Laserlicht die Ablagerungen im Umfangsbereich des Gefäßes trifft, welches durch die Laserstrahlung kanalisiert werden soll. Man hat den Durchmesser der ringförmigen Lichtleiteranordnung daher im allgemeinen derart gewählt, daß die Lichtleiter auf einem Umfangskreis lagen, der in etwa den Ablagerungen entsprach. Ein solcher Katheter wird dann bis zur Verengungsstelle in das zu behandelnde Gefäß eingeführt und kann nur insoweit weiter vorgeschoben werden, wie die Ablagerungen an der Außenwand des Gefäßes durch die Behandlung entfernt worden sind. Um den Katheter bei fortschreitender Behandlung und weiteren Vorschubversuchen in die richtige Richtung zu bringen, benutzt man den im Katheter vorgesehenen Innenkanal beziehungsweise das Lumen außer als Durchflußkanal für Spülflüssigkeit auch zum Einschieben eines dünneren Führungsdrahtes, der sich bereits durch die noch nicht behandelten Verengungsstellen vorschieben läßt, und der dann als Leitbahn für das weitere Vortreiben des Ringkatheters im Zuge der Behandlung dient.

Nun ist es aber nicht immer möglich und daher wünschenswert, Ringkatheter mit einem Durchmesser zu verwenden, der bereits weitgehend dem Innendurchmesser des Gefäßes entspricht, welches rekanalisiert werden soll. Insbesondere für das Vortreiben eines Laserkatheters in Regionen geringeren Gefäßdurchmessers, beispielsweise in der Unterschenkeletage des Menschen oder in Herzkoronarien ist es wünschenswert, Lichtleitkatheter zu verwenden, die einen möglichst geringen Durchmesser aufweisen, der möglichst noch unter French 5 liegen soll.

Der DE-OS 3 739 965 läßt sich beispielsweise entnehmen, daß je nach optischer Austrittscharakteristik des distalen Endes des Lichtleiterkatheters die Energiedichte der Laserstrahlung schon in geringem Abstand von der Austrittsfläche soweit abnimmt, daß sie für eine effektive Entfernung der Gefäßablagerungen nicht mehr ausreicht. Dies kann insbesondere dann eintreten, wenn ein verhältnismäßig dünner Ringkatheter verwendet wird, die zu entfernenden Ablagerungen, beispielsweise in einem erweiterten Teil des Gefäßes, asymmetisch einseitig angeordnet sind oder außerhalb des Durchmesserbereiches des Katheters liegen. In solchen Fällen kann es unvermeidlich werden, den eingesetzten Katheter, je nach Erfordernis, durch einen solchen mit größerem oder kleinerem, dem jeweiligen Lumen des Gefäßes angepaßten Innendurchmesser zu ersetzen.

Der Erfindung liegt daher die Aufgabe zugrunde, ein Kathetersystem der eingangs bezeichneten Art derart weiterzubilden, daß der Durchmesser des Lichtleiterringkatheters möglichst gering gehalten werden kann, die Laserstrahlung jedoch bei Bedarf so gerichtet werden kann, daß mit ihr wirksam auch Ablagerungen beseitigt werden können, die im wesentlichen außerhalb des Durchmessers des Ringkatheters liegen.

Diese Aufgabe wird erfindungsgemäß durch ein Kathetersystem gelöst, welches die Merkmale des kennzeichnenden Teiles des Patentanspruches 1 aufweist.

Durch elastisch aufweitbare Verbindungsstücke, vorzugsweise aus Polymethylsiloxan, zwischen den Lichtleitern des Lichtleiterkranzes kann erreicht werden, daß die Lichtleiter nicht nur durch parallel versetzendes Aufweiten des Kranzes eine ringförmige Austrittsfläche größeren Durchmessers einnehmen, es ist durch graduell zunehmende Elastizität des verbindenden Materials zwischen den Lichtleitern zum distalen Ende des Katheters hin beziehungsweise durch Beschränkung der elastischen Ausbildung nur auf einen kleinen Längenabschnitt vor dem Austrittsende des Katheters möglich, den Lichtleiterkranz divergent aufzuweiten, so daß die aus den Lichtleitern austretende Laserstrahlung jeweils von der Achse des Katheters weg zur Gefäßwand gerichtet wird.

Da ein einzelner Lichtleiter im allgemeinen jedoch nicht genügend Laserenergie transportiert, um eine Ablösung von Gefäßablagerungen zu bewirken, kann eine gleichmäßige Aufweitung des Lichtleiterkranzes am distalen Ende des Katheters dazu führen, daß durch die Vergrößerung des Abstandes der einzelnen Lichtleiter zueinander die Energiedichte der Strahlung in unerwünschter Weise abnimmt. In bevorzugter Ausführungsform der Erfindung sind die Lichtleitfasern über den Umfang des Ringkatheters am distalen Ende daher gruppenweise fest miteinander verbunden, wobei die elastische Aufweitbarkeit allein auf Übergangsbereiche zwischen diesen Gruppen beschränkt ist. Dadurch läßt sich erreichen, daß eine Gruppe von mehreren Lichtleitfasern, die ihren gegenseitigen Abstand zueinander im wesentlichen beibehalten, in Richtung auf die Gefäßwand hin abgebogen werden kann, wobei dann, bezogen auf diese Lichtleitergruppe, die erforderliche Energiedichte erhalten bleibt.

Da die Gefäßablagerungen häufig asymmetrisch auftreten und daher vielfach nur spezielle Bereiche der Gefäßinnenwand zu behandeln sind, ist es ohnehin im allgemeinen nicht immer erforderlich, den gesamten Lichtleiterkranz mit Laserlicht zu beaufschlagen. Zu diesem Zweck sieht die Erfindung weiterhin vor, daß am proximalen Eintrittsende des Ringkatheters die Lichtleitfasern zu bestimmten Gruppen zusammengefaßt sind, welche sich selektiv mit Laserlicht beaufschlagen lassen. Diese Gruppen sind natürlich bevorzugt mit den Gruppen fest miteinander verbundener Lichtleitfasern am distalen Ende des Katheters identisch. Um möglichst geringe Einstrahlungsverluste zu haben und die Fassung der Lichtleitfasern am proximalen Ende nicht durch Laserstrahlung zu zerstören, die nicht in die Lichtleitfasern eingekoppelt wird, sind die Fasern am proximalen Ende des Katheters vorzugsweise in hexagonal dichtester Packung angeordnet, wobei diese Gruppen von Lichtleitfasern zusammenhängende Flächenbereiche der hexagonalen Packung bilden. Diese Flächenbereiche können beispielsweise parallele Flächenstreifen der hexagonalen Packung sein. Solche Streifen lassen sich auf einfache Weise durch parallele Steckblenden oder dergleichen ausblenden.

Die Aufweitung des Lichtleitfaserkranzes am distalen Ende des Katheters kann beispielsweise durch eine Kammer erfolgen, die sich mit einem Druckfluid beaufschlagen läßt. Es ist auch möglich, über den Umfang des Katheters verteilt Teilkammern vorzusehen, mit deren Hilfe eine Aufweitung des Ringkatheters nur in bestimmten Umfangsbereichen vornehmbar ist, in denen dann zusätzlich die Lichtleitfasern selektiv mit Laserlicht beaufschlagbar sind.

Da die betroffenen Katheter jedoch, wie oben erwähnt, im allgemeinen bereits aus anderen Gründen mit einem Innenkanal versehen sind, sieht die Erfindung in bevorzugter Ausführungsform vor, ein Miniaturdilatationskatheter vorzusehen, welches bis zum distalen Ende in den Innenkanal des Ringkatheters einführbar und dort expandierbar ist, um den Lichtleiterkranz insgesamt oder in gezielter radialer Richtung aufzuweiten.

Es gibt verschiedene Möglichkeiten, die Behandlung nur eines bestimmten Innenwandbereiches durch selektive Beaufschlagung von Lichtleitfasern zu bestimmen und zu beobachten. So können einzelne der Lichtleitfasern des Kranzes zur Beleuchtung der Behandlungsstelle benutzt werden, die dann über ein in den Innenkanal des Katheters eingeführtes Endoskop beobachtet werden kann. Auch läßt sich durch Analyse einer vom Behandlungsort reflektierten Strahlung erkennen, ob tatsächlich Ablagerungen oder Gewebe der Gefäßwand im vorgesehen Strahlungsbereich liegen. Derartige Methoden sind jedoch zum Teil bekannt und sind nicht Gegenstand dieser Erfindung. Auch wenn die Aufweitbarkeit des Lichtleiterkranzes am distalen Ende des Katheters nur auf einen bestimmten Umfangsbereich beschränkt ist, so läßt sich dieser Bereich im allgemeinen in die gewünschte Behandlungsposition bringen, indem man auf den Katheter erforderlichenfalls unter gewissem axialen Hin- und Herbewegen am proximalen Ende eine drehende Kraft ausübt.

Durch die erfindungsgemäße Ausbildung des Kathetersystems ist es möglich, auch bei Verwendung eines sehr dünnen Laser-Ringkatheters Bereiche einer Gefäßwand durch Laserstrahlung zu behandeln, die in einem Abstand von der Achse des Katheters liegen, der größer sein kann als der Radius des Ringkatheters. Auch bei Ablagerungen, die im Umfangsbereich des Katheterendes liegen, ist die erfindungsgemäße Ausführung von Vorteil, da durch die divergente Ablenkbarkeit der Enden der Lichtleitfasern eine höhere Energiedichte für die Ablösung der Ablagerungen erzielt werden kann, da unter Umständen mit einem steileren Einfallwinkel der Laserstrahlung auf die zu behandelnde Stelle gearbeitet werden kann.

Selbst bei den Gelegenheiten, wo Laserkatheter in Bereiche geringer Gefäßdurchmesser vorgetrieben werden müssen, läßt sich dies mit der erfindungsgemäßen Ausführungsform infolge der elastischen Verbindungen zwischen den Lichtleitfasern bzw. Fasergruppen leicht verwirklichen. Dabei kann, insbesondere nach mehrmaligem vorsichtigen Hin- und Herfahren des Katheters, das distale Ende problemlos an die Gefäßablagerungen herangeführt werden. Ein Katheterwechsel, wie in solchen Fällen nach dem bisherigen Stand der Technik meistens erforderlich, entfällt.

Im folgenden werden Ausführungsbeispiele der Erfindung anhand der beigefügten Zeichnungen noch näher erläutert.

Es zeigen:

Figur 1 eine schematische Darstellung der Zuordnung der Fasergruppen am distalen Ende zu den Fasergruppen in hexagonal dichtester Packung am proximalen Ende des Katheters,

Figur 2 eine schematische Darstellung der Steckblenden zur Ausblendung der entsprechenden Flächenstreifen auf der hexagonal dichtesten Faserpackung am proximalen Ende des Katheters, und

Figur 3 ein Längsschnitt durch das distale Ende des Ringkatheters mit eingeführtem Miniaturdilatationskatheter und expandiertem Lichtleiterkranz.

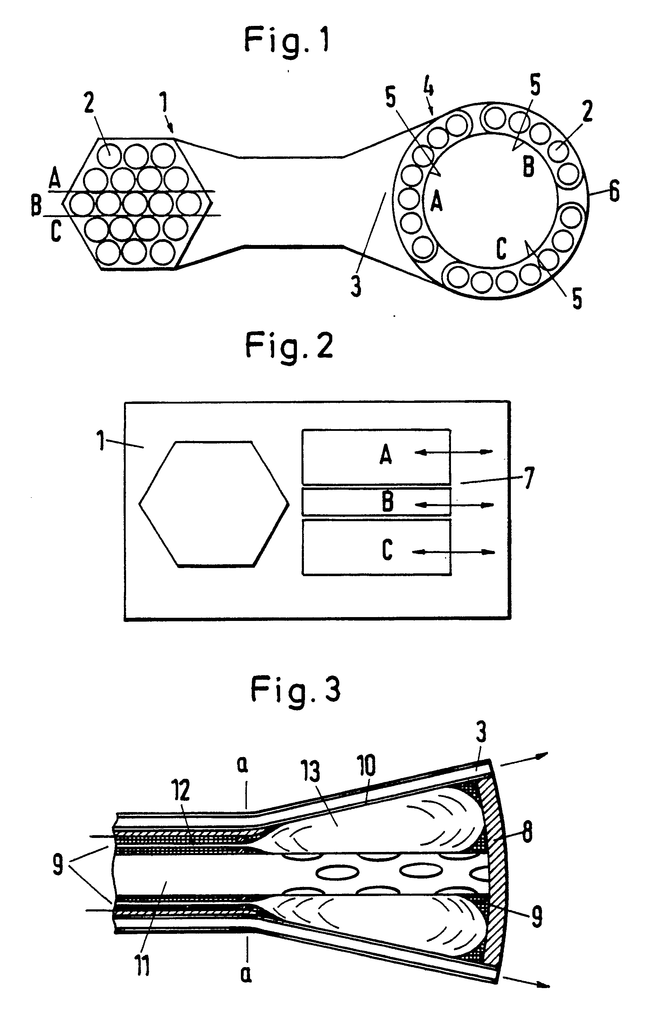

In Figur 1 sind das proximale und distale Ende schematisch dargestellt. Das proximale Ende 1 besteht aus der hexagonal dichtesten Packung der einzelnen Lichtleitfasern 2. Im Lichtleiterkranz 3 am distalen Ende 4 sind die Lichtleitfasern 2 jeweils in Umfangsgruppen 5 fest miteinander verbunden und die einzelnen Gruppen A, B, C hängen ihrerseits über elastisch verformbare Zwischenbereiche 6 miteinander zusammen. Den jeweiligen Umfangsgruppen 5 am distalen Ende, welche aus fest miteinander verbundenen Lichtleitfasern 2 bestehen, entsprechen am proximalen Ende die jeweiligen parallelen Flächenbereiche A, B, C der hexagonalen Packung.

Diese Flächenbereiche A, B, C können mit den in Figur 2 wiedergegebenen Steckblenden 7 einzeln oder jeweils zwei davon gleichzeitig ausgeblendet werden, so daß am distalen Ende jeweils nur eine oder zwei Fasergruppen A, B, C mit Laserlicht beaufschlagt werden können.

In Figur 3 ist in den Innenkanal 8 ein Miniaturballondilatationskatheter 9 eingeführt. Zwischen dessen Ummantelung 10 und dessen Innenkanal 11 sind Zuführungskanäle 12 für ein Druckfluid angebracht, die an Punkt a jeweils in einen Ballon 13 münden. Unter Druckbeaufschlagung leiten die Ballons, vorzugsweise mindestens drei, den Lichtleiterringkranz 3 am distalen Ende über den Längsabschnitt ab Punkt a bis zum lichtaustrittsseitigen Ende des Katheters unter Verformung der elastischen Zwischenbereiche 6 auf.

In einer anderen bevorzugten Ausführungsform, die hier nicht abgebildet ist, können die Zuführungskanäle für Druckfluid und die Anschwellvorrichtung im distalen Endbereich direkt im Faserkranz des Katheters integriert werden, wobei die quellbaren Druckkammern direkt in die elastischen Verbindungsstücke 6 im distalen Endbereich zu liegen kommen. Unter Druckbeaufschlagung von jeweils zwei benachbarten Druckkammern kann somit der dazwischenliegende Bereich mit fest miteinander verbundenen Lichtleitfasern selektiv in Richtung auf die Gefäßwand abgebogen werden.

**Ansprüche**

1. Kathetersystem zur Übertragung von Laserstrahlung zu Behandlungszwecken, insbesondere im Gefäßsystem des menschlichen Körpers mit einem im Katheterwandaufbau angeordneten Kranz von Lichtleitern, an deren proximalen Enden Mittel zur Einkoppelung von Laserlicht vorgesehen sind, dadurch gekennzeichnet, daß der Lichtleiterkranz (3) am distalen Lichtaustrittsende (4) des Katheters unter Vergrößerung seines Umfangs aufweitbar ausgebildet ist, wobei die Vergrößerung des Umfanges durch Vergrößern des Abstandes zwischen den einzelnen Lichtleitfasern (2) des Kranzes oder zwischen bestimmten Umfangsgruppen (5) von Lichtleitfasern des Kranzes erfolgen kann.

2. Kathetersystem nach Anspruch 1, dadurch gekennzeichnet, daß die Lichtleiter (2) beziehungsweise Gruppen von Lichtleitern (5) des Lichtleiterkranzes (3) am distalen Ende (4) des Kathetersystems durch ein elastisch dehnbares Material (6), vorzugsweise Polymethylsiloxan, miteinander verbunden sind.

3. Kathetersystem nach Anspruch 1 oder 2, dadurch gekennzeichnet, daß zum Aufweiten des Lichtleiterkranzes (3) im distalen Ende (4) des Katheters eine durch den Katheter mit einem Druckfluid beaufschlagbare Dehnkammereinrichtung vorgesehen ist.

4. Kathetersystem nach Anspruch 3, dadurch gekennzeichnet, daß die Dehnkammereinrichtung getrennt beaufschlagbare, in unterschiedlichen radialen Richtungen wirkende Teilkammern aufweist.

5. Kathetersystem nach Anspruch 1 oder 2 mit einem vom die Lichtleiter enthaltenden Wandaufbau umgebenen, durchgehenden Innenkanal, dadurch gekennzeichnet, daß zum Aufspreitzen des distalen Endes des Lichtleiterkranzes ein in den Innenkanal (8) einführbarer und im distalen Ende des Katheters positionierbarer Miniaturdilatationskathether (9) vorgesehen ist.

6. Kathetersystem nach einem der Ansprüche 1 bis 5, dadurch gekennzeichnet, daß die Mittel zum Einkoppeln von Laserlicht in die proximalen Enden der Lichtleiter selektiv betätigbare Bereichsblenden (7) aufweisen, mit deren Hilfe jeweils bestimmte Gruppen von Lichtleitern mit Laserlicht beaufschlagbar sind, und daß diese Gruppen mit den Umfangsgruppen (5) von Lichtleitfasern am distalen Ende des Katheters identisch sind.

7. Kathetersystem nach Anspruch 6, dadurch gekennzeichnet, daß die Lichtleiter am proximalen Ende zur Einkoppelung des Laserlichtes in hexagonal dichtester Packung (1) zusammengefaßt sind, und daß diese Gruppen von Lichtleitern zusammenhängende Flächenbereiche der hexagonalen Packung bilden.

# Fig. 1

# Fig. 2

# Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 790 310 (GINSBURG et al.) * Spalte 2, Zeile 45 - Spalte 3, Zeile 4 * | 1-5 | A 61 B 17/22 |
| Y | | 6,7 | |
| Y | US-A-4 296 319 (FRANKS et al.) * Spalte 3, Zeile 67 - Spalte 4, Zeile 23; Figur 3 * | 6,7 | |
| A | EP-A-0 217 165 (FOX) * Seite 18, Zeilen 18-27; Figuren 3A,B * | 1,4 | |

|  | RECHERCHIERTE SACHGEBIETE (Int. Cl.5) |
|---|---|
|  | A 61 B |

Der vorliegende Recherchenbericht wurde für alle Patentansprüche erstellt

| Recherchenort | Abschlußdatum der Recherche | Prüfer |
|---|---|---|
| DEN HAAG | 13-06-1990 | MOERS R.J. |